(19) Europäisches Patentamt
European Patent Office
Office européen des brevets

(11) EP 0 595 567 B1

(12) EUROPEAN PATENT SPECIFICATION

(45) Date of publication and mention of the grant of the patent:
**06.03.1996 Bulletin 1996/10**

(51) Int Cl.[6]: **C07C 43/04**, C07C 41/09

(21) Application number: **93308464.2**

(22) Date of filing: **25.10.1993**

# (54) Synthesis of alkyl-t-alkyl ether using beta-zeolite catalysts

Synthese von Alkyl-tert-alkylethern unter Verwendung von Betazeolithkatalysatoren

Synthèse d'éthers alkyl tertio-alkyl utilisant des catalyseurs bêta-zéolitiques

(84) Designated Contracting States:
**BE DE FR GB NL**

(30) Priority: **28.10.1992 US 967479**

(43) Date of publication of application:
**04.05.1994 Bulletin 1994/18**

(73) Proprietor: **TEXACO CHEMICAL INC.**
**Houston, Texas 77056 (US)**

(72) Inventors:
- **Knifton, John Frederick**
  **Austin, TX 78750 (US)**
- **Dai, Pei-Shing Eugene**
  **Port Arthur, TX 77642 (US)**

(74) Representative: **Green, Mark Charles et al**
**Urquhart-Dykes & Lord,**
**91 Wimpole Street**
**London W1M 8AH (GB)**

(56) References cited:
**EP-A- 0 333 078**
**EP-A- 0 546 695**
**US-A- 5 081 318**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 595 567 B1

## Description

This invention relates to the synthesis of alkyl-t-alkyl ether using selected zeolite catalysts.

In particular, this invention concerns an improved process for preparing methyl tertiary-butyl ether (MTBE) along with isobutylene and, optionally, diisobutylene by the reaction of tertiary butanol and methanol in the presence of a catalyst comprising β-zeolite or β-zeolite modified with a metal selected from the group consisting of Groups IB, VB, VIB, VIIB and VIII of the Periodic Table as defined in the Condensed Chemical Dictionary, Tenth Edition, page 789. Metals which work well include transition metals found in Row 1 of Groups VIB, VIIB and VIII, particularly iron, manganese and chromium. The invention is especially advantageous in that the multimetal-modified zeolites exhibit high activity during alkyl-t-alkyl ether synthesis from primary alcohol and tertiary alkyl alcohol, e.g. methyl t-butyl ether synthesis from methanol plus t-butanol, extended catalyst life with crude feedstocks and, additionally, exhibit concurrent quantitative peroxide decomposition of, for example di-t-butyl peroxide (DTBP), in the crude alcohol feedstock.

Alkyl tertiary-alkyl ethers prepared by this invention, particularly methyl tert-butyl ether, are useful as a blending component in high octane gasoline.

It is known to those skilled in the art that ethers, including unsymmetrical ethers, may be prepared by reacting an alcohol with another alcohol to form the desired product. The reaction mixture, containing catalyst and/or condensing agent may be separated and further treated to permit attainment of the desired product. Such further treatment commonly includes one or more distillation operations.

Methyl tert-butyl ether is finding increasing use as a blending component in high octane gasoline as the current gasoline additives based on lead and manganese are phased out. Currently all commercial processes for the manufacture of methyl tert-butyl ether are based upon the liquid-phase reaction of isobutylene and methanol (Eq. 1), catalyzed by a cationic ion-exchange resin (see, for example: Hydrocarbon Processing, Oct. 1984, p. 63; Oil and Gas J., Jan. 1, 1979, p. 76; Chem. Economics Handbook-SRI, Sept. 1986, p. 543-7051P). The cationic ion-exchange resins used in MTBE synthesis normally have the sulphonic acid functionality (see: J. Tejero, J. Mol. Catal., 42 (1987) 257; C. Subramamam et al., Can. J. Chem. Eng., 65 (1987) 613).

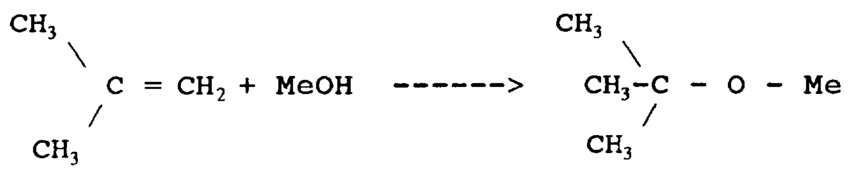

(Eq. 1)

With the expanding use of MTBE as an acceptable gasoline additive, a growing problem is the availability of raw materials. Historically, the critical raw material is isobutylene (Oil and Gas J., June 8, 1987, p. 55). It would be advantageous, therefore, to have a process to make MTBE that does not require isobutylene as a building block. It would be advantageous to have an efficient process for making MTBE by reaction of methanol with tertiary butyl alcohol, since t-butanol (tBA) is readily available commercially through isobutane oxidation.

Japanese Patent 0007432 teaches the use of zeolites to make dialkyl ethers containing primary or secondary alkyl groups. The zeolites have a porous structure and are represented by:

$$M_{2/n}O \cdot Al_2O_3 \cdot xSiO_2 \cdot yH_2O$$

where M is an alkali metal or alkaline earth metal cation or organic base cation, n is the valence of the cation and x and y are variables.

US-A-4,058,576 (Chang et al) teaches the use of (pentasil-type) aluminosilicate zeolites, such as ZSM-5, having a pore size greater than 5 angstrom units and a silica-to-alumina ratio of at least 12, to convert lower alcohols to a mixture of ethers and olefins.

US-A-5214217 (Texaco) discloses a method for preparing methyl tertiary butyl ether by reacting butanol and methanol in the presence of a catalyst comprising a super-acid alumina or a faujasite-type zeolite.

In US-A-5,081,318 (Texaco), a Y-type zeolite modified with fluorosulfonic acid is disclosed.

One of the earliest disclosures of β-zeolite was in US-A-3,308,069 (1967) (Wadinger et al).

J.B. Higgins, et al of Mobil Research and Development published an article in ZEOLITES, 1988, Vol. 8, November, 446-452 titled "The Framework Topology of Zeolite Beta." In the article Higgins et al. disclose what is known about the framework topology of β-zeolite. The information has been determined using a combination of model building, distance-least-square refinement and powder pattern simulation.

In an article titled "Cumene Disproportionation over Zeolite β I. Comparison of Catalytic Performances and Reaction Mechanisms of Zeolites," Applied Catalysis, 77 (1991) 199-207, Tseng-Chang Tsai, Chin-Lan Ay and Ikai Wang disclose a study demonstrating that cumene disproportionation can be applied as a probe reaction for zeolite structure. It is revealed that β-zeolite would have application potential in the production of diisopropylbenzene for reasons of activity,

selectivity and stability.

In a second part of the article, "II. Stability Enhancement with Silica Deposition and Steam Pretreatment", Ibid, pp. 209-222, Tsai and Wang disclose their development of two methods to improve the stability of β-zeolite, silica deposition and steam pretreatment.

Patents in the art which employ β-zeolite relate mainly to dewaxing, and cracking of hydrocarbon feedstock.

US-A-4,419,220 (Mobil) discloses a process for dewaxing a hydrocarbon feedstock containing straight chain paraffins which comprises contacting the feedstock with a β-zeolite catalyst having a Si:Al ratio of at least 30:1 and a hydrogenation component under isomerization conditions.

EP-A-0 094 82 (Mobil), discloses simultaneous catalytic hydrocracking and hydrodewaxing of hydrocarbon oils with β-zeolite.

EP-A-0 095 303 (Mobil) discloses dewaxing distillate fuel oils by the use of β-zeolite catalysts which, preferably have a silica:alumina ratio over 100:1. Ratios as high as 250:1 and 500:1 are disclosed as useful.

US-A-4,518,485 (Mobil) discloses a process for dewaxing a hydrocarbon feedstock containing paraffins selected from the group of normal paraffins and slightly branched paraffins and sulfur and nitrogen compounds where, after conventionally hydrotreating the feedstock to remove sulfur and nitrogen, the hydrotreated feedstock is dewaxed by contacting the feedstock with a catalyst comprising β-zeolite having a silica/alumina ratio of at least 30:1.

US-A-4,740,292 (Mobil) discloses a catalytic cracking process which comprises cracking a hydrocarbon feed in the absence of added hydrogen with a cracking catalyst comprising a β-zeolite component and a faujasite component comprising at least one crystalline aluminosilicate of the faujasite structure, the weight ratio of the faujasite component to the β-zeolite component being from 1:25 to 20:1.

EP-A-0333078 (Texaco) discloses a method for the one-step synthesis of methyl t-butyl ether, wherein t-butanol is reacted with methanol in a reaction zone in the presence of a catalyst to provide methyl-tert-butyl ether and the improvement of accomplishing the reaction in one-step which comprises using a catalyst selected from the group consisting of acidic aluminas, acidic silicas, or combinations thereof, and zeolites containing alumina or silica, and acidic clay mineral catalysts.

Some of the limitations present in the MTBE catalyst systems described above include loss of activity at temperatures above 120°C, deactivation due to the presence of peroxides in the feedstock, lower than desirable selectivity and the requirement of multiple steps to accomplish the synthesis and separation of the product.

It would represent a distinct advance in the art if tertiary butanol, instead of isobutylene and methanol could be reacted to form MTBE in one-step over a β-zeolite or modified β-zeolite catalyst which exhibited the ability to withstand elevated temperatures, exhibited an extended useful life and allowed for good selectivity for the desired product even in the presence of peroxides. It would also be very useful if crude product phase separation were possible. In addition, it would be very useful in the art if a catalyst which allowed for MTBE plus isobutylene cosynthesis exhibited high levels of selectivity and stability for up to 2000 hours using crude tBA/MeOH feedstocks.

In accordance with certain of its aspects, the novel method of this invention for preparing alkyl tert-alkyl ether from tertiary alkyl alcohol and primary alcohol, e.g. preparing methyl tert-butyl ether (MTBE) from tertiary butyl alcohol (t-butanol or tBA) and methanol (MeOH) in one-step comprises reacting tertiary alkyl alcohol and primary alcohol in the presence of a catalyst comprising a β-zeolite or multimetal-modified β-zeolite at an elevated temperature and moderate pressure. Examples demonstrate particularly the effectiveness of an iron, copper, nickel, manganese and chromium-modified β-zeolite.

Typically t-butanol conversion levels of approximately 70% are achieved at 120°C using near stoichiometric feedstocks.

Preparation of the product of this invention may be carried out typically by reacting tertiary butyl alcohol and methanol in the presence of an etherification catalyst. The etherification may be carried out in one-step and the catalyst preferably comprises β-zeolite alone or modified with one or more metals selected from the group consisting of Group IB, VB, VIB, VIIB or VIII of the Periodic Table.

The reaction can be represented by the following:

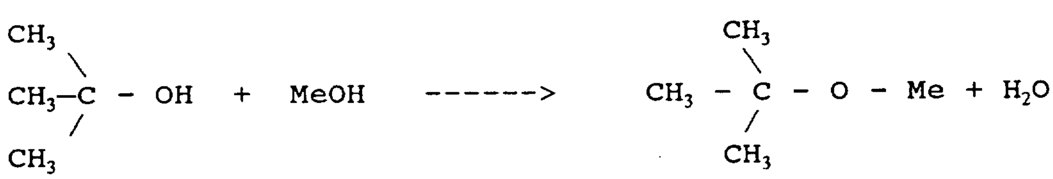

(Eq. 2)

Generally the methanol and t-butanol coreactants may be mixed in any proportion in order to generate the desired methyl t-butyl ether, but preferably the molar ratio of methanol to t-butanol in the feed mixture should be between 10:1 and 1:10, if the yield of desired MTBE is to be maximized. In order to achieve maximum selectivity to MTBE, and optimum conversion per pass, an excess of methanol in the liquid feed is desirable. The most preferred methanol-to-tertiary

butanol molar ratio is from 1:1 to 5:1.

In certain circumstances, it may be particularly desirable that the tBA conversion be high enough (e.g. 70% or greater), such that the crude product mix phase separates into an isobutylene-MTBE product-rich phase and a heavier aqueous methanol phase. Preferably such a product phase separation would be achieved at as low an etherification temperature as possible, but it is particularly observed in the range 140°-200°C.

The synthesis of Eq. 2 can also be conducted where the t-butanol and methanol reactants are mixed with certain other components including water, ketones such as acetone ($Ac_2O$) and methyl ethyl ketone (MEK), peroxides and hydroperoxides such as di-t-butyl peroxide (DTBP) and allyl t-butyl peroxide (ATBP), and t-butyl hydroperoxide (TBHP), as well as esters such as t-butyl formate (TBF). Typically each of said classes of components makes up less than 10% of the total feed mixture.

It has been discovered in the instant invention that the β-zeolites and the multimetal-modified β-zeolite catalysts herein disclosed function to exhibit concurrent quantitative decomposition of peroxide in the alcohol feedstock in addition to converting tertiary butanol plus methanol to MTBE. This constitutes an important advantage in a commercial setting.

The instant one-step process may also be applied to the preparation of alkyl tertiary alkyl ethers other than MTBE. For example, said process may be applied to the reaction of a $C_1$-$C_6$ primary alcohol such as methanol, ethanol, n-propanol and n-hexanol with a $C_4$-$C_{10}$ tertiary alcohol such as, for example, tertiary butanol and tertiary amyl alcohol. Reaction of methanol with tertiary amyl alcohol (2-methyl-2-butanol) would then yield methyl tertiary amyl ether (TAME), while reaction of ethanol with t-butanol would yield ethyl t-butyl ether (ETBE). Alternatively a mixture of alcohols, e.g., a mixture of $C_1$-$C_5$ alcohols, could be reacted to give a mixture of alkyl tert-alkyl ethers.

In the modified catalyst of the instant invention good results were realized using certain crystalline aluminosilicate zeolites as catalysts for the reaction represented in Eq. 2. Particularly effective were the isostructural group of β-zeolites.

β-zeolite was first synthesized at the Mobil Research and Development Laboratories. It exhibited improved thermal and acid stability over previously synthesized zeolites, Higgins et al., supra, p. 446.

The composition of β-zeolite is described in US-A-3,308,069; 4,419,220; 4,518,485 and 4,740,292. In those references, β-zeolite is generally described as follows:

β-zeolite is a crystalline aluminosilicate having a pore size greater than 5 Angstroms. The composition of the zeolite, as described in US-A-3,308,069, in its as synthesized form may be expressed as follows:

$$[XNa(1.0\pm0.1\text{-}X)TEA]AlO_2.YSiO_2\cdot WH_2O$$

where X is less than 1, preferably less than 0.7; TEA represents the tetraethylammonium ion; Y is greater than 5 but less than 100; and W is up to about 60 (it has been found that the degree of hydration may be higher than originally determined, where W was defined as being up to 4), depending on the degree of hydration and the metal cation present. The TEA component is calculated by differences from the analyzed value of sodium and the theoretical cation to structural aluminum ratio of unity.

As discussed in the J. B. Higgins, et al. reference, Supra, p. 446, the first clues to the crystal structure of β-zeolite were evidenced from chemical and physical property measurements. Ion-exchange isotherms of Na-β-zeolite at 25°C indicated that cations as large as tetraethylammonium ($TEA^+$) exchanged completely into the pore system. This behavior suggests that β-zeolite contains at least 12-membered rings opening into channels, because $TEA^+$ is too large to exchange through 10-membered rings such as those in ZSM-5. The complete exchange of cations in β-zeolite indicated the presence of channels instead of cages, because it is not possible to remove all the cations from cage structures such as Na faujasite. Additional evidence was obtained from organic sorption data and density measurements. Cyclohexane sorption of 14.6-19.4 wt% and a measured density of 1.61 g/$cm^3$ ruled out undimensional pore systems such as those in ZSM-12, ZSM-22, ZSM-23 and ZSM-48. Structural similarities among β-zeolite, mordenite and ZSM-12 were suspected because all three may be synthesized in $Na^+$-$TEA^+$ systems from highly siliceous batch compositions. Further, β-zeolite is easily synthesized in the $SiO_2/Al_2O_3$ range of 30-50. This lies between $TEA^+$ mordenite (typically 10-30) and ZSM-12 (typically, >60), suggesting the β-zelite framework contains large fractions of both 4- and 5-membered rings.

In the Tsai and Wang reference, Supra, part II, p. 209, stability enhancement is discussed. Two methods, silica deposition and steam pretreatment, have been developed to substantially improve β-zeolite stability.

At comparable conversion levels, the alpha value decreases from 0.23 for unmodified β-zeolite to 0.09 after mild steam pretreatment. Where β-zeolite is steam pretreated, apparently acid strength of the zeolite is enhanced and the yield of aromatics is increased, Ibid, p. 213.

Where silica is deposited on the β-zeolite, the pore openings are narrowed and the selectivity for para-diisopropyl benzene is enhanced. On p. 215, Ibid, it is stated that maximum stabilization was obtained around 0.10g $SiO_2$ $g_{cat}^{-1}$. According to this reference the activity of β-zeolite completely disappeared at a silica deposition of 0.21g $SiO_2$ $g_{cat}^{-1}$. It is stated that the best stability enhancement with silica deposition, obtaining an alpha value of 0.08, is less significant than that with steam pretreatment, wherein a value of 0.01 is obtained, however the excellent disproportionation selectivity of β-zeolite is little affected by silica deposition.

Ibid, p. 215, it is stated that β-zeolite has two types of three dimensional pore openings, the linear and the tortuous

channel. The former has pore openings of 7.5Å x 5.7Å and the latter has pore openings of 6.5Å x 5.6Å. When silica, for example, is deposited on β-zeolite, the pore opening was narrowed or blocked by the deposited silica. It was concluded that silica deposition selectively removes strong acid sites and increases the population of medium acid sites.

In the fully base-exchanged form, β-zeolite has the composition:

$$[(X/n)M(1\pm0.1-X)H]AlO_2\cdot YSiO_2\cdot WH_2O$$

where X, Y and W have the values listed above and n is the valence of the metal M. This form of the zeolite may be converted partly to the hydrogen form by calcination, e.g. at 200°C to 900°C or higher. The completely hydrogen form may be made by ammonium exchange followed by calcination in air or an inert atmosphere such as nitrogen, see US-A-4,419,220.

β-zeolite is characterized by the following X-ray diffraction pattern:

d Values of Reflection in β-zeolite

11.40 ± 0.2
7.40 ± 0.2
6.70 ± 0.2
4.25 ± 0.1
3.97 ± 0.1
3.00 ± 0.1
2.20 ± 0.1

The preferred forms of β-zeolite are the highly acidic, high silica forms, having silica-to-alumina mole ratio of at least 10:1, and preferably in the range of 10:1 to 50:1 in the as-synthesized form. A narrower range of 20:1 to 30:1 is preferred and the β-zeolite powders demonstrated in the examples possess $SiO_2/Al_2O_3$ ratios of about 23:1 to 26:1. It has been found, in fact, that β-zeolite may be prepared with silica-to-alumina mole ratios above the 200:1 maximum specified in US-A-3,308,069 and these forms of the zeolite may perform well in the process. Ratios of 50:1, or even higher, may be used where available.

The silica-to-alumina ratios referred to in this specification are the structural or framework ratios, that is, the ratio of the $SiO_4$ to the $AlO_4$ tetrahedra, which together constitute the structure of which the zeolite is composed. It should be understood that this ratio may vary from the silica-to-alumina ratio determined by various physical and chemical methods. For example, a gross chemical analysis may include aluminum which is present in the form of cations associated with the acidic sites on the zeolite, thereby giving a low silica-to-alumina ratio. Similarly, if the ratio is determined by the thermogravimetric analysis (TGA) of ammonia desorption, a low ammonia titration may be obtained if cationic aluminum prevents exchange of the ammonium ions onto the acidic sites. These disparities are particularly troublesome when certain treatments, such as the dealuminization method described below which result in the presence of ionic aluminum free of the zeolite structure, are employed. Due care should therefore be taken to ensure that the framework silica-to-alumina ratio is correctly determined.

The silica-to-alumina ratio of the zeolite may be determined by the nature of the starting materials used in its preparation and their quantities relative one to another. Some variation in the ratio may therefore be obtained by changing the relative concentration of the silica precursor relative to the alumina precursor, but definite limits in the maximum obtainable silica-to-alumina ratio of the zeolite need be observed. For β-zeolite, this limit is usually about 100:1 (although higher ratios may be obtained) and for ratios above this value, other methods are usually necessary for preparing the desired high silica zeolite. This method generally comprises contacting the zeolite with an acid, preferably a mineral acid such as hydrochloric acid. The dealuminization proceeds readily at ambient and mildly elevated temperatures and occurs with minimal losses in crystallinity to form high silica forms of β-zeolite with silica-to-alumina ratios of at least 100:1, with ratios of 200:1 or even higher being readily attainable.

Particularly effective in the subject synthesis of MTBE are the β-zeolites modified with multiple metals.

Illustrative of suitable β-zeolites for the practice of this invention include Valfor C806β, Valfor CP815β and Valfor C861. Valfor® is the registered trademark of the PQ Corporation. Valfor® C806β zeolite is β-zeolite powder in template cation form. It is a high silica shape selective zeolite which contains the organic template used in the crystallization step, having been isolated after filtration and washing of the synthesis product. C806β has a $SiO_2/Al_2O_3$ molar ratio of 23-26; the crystal size is 0.1-0.7 μm; the surface area after calcination is about 700-750 $m^2/g$; the cyclohexane adsorption capacity after calcination is 19-24g/100g; $Na_2O$ content is about 0.5-1.0% by weight anhydrous; and, the organic content is about 11-13% by weight, on a water-free basis.

Valfor® C815β zeolite is a calcined β-zeolite powder in hydrogen, sodium form. It is similar to C806β except the product has been calcined to decompose the organic template. C815β is a high silica, shape selective aluminosilicate with a large pore diameter. C815β also has a $SiO_2/Al_2O_3$ molar ratio of about 23-26; the crystal size, surface area, cyclohexane adsorption capacity and $Na_2O$ are all within the same ranges as given for C806β,

Valfor® C861β is an extrudate made of 80% C815β powder and 20% alumina powder.

The metals useful for modifying the zeolite in the instant invention comprise those from Group IB, VB, VIB, VIIB and VIII of the Periodic Table, including said transition metals. Preferred metals are those found in Row 1 of Groups IB, VIB and VIII of the Periodic Table and include copper, chromium, manganese, iron and nickel. Especially good results were observed using combinations of iron, manganese and chromium or combinations of nickel, copper and chromium, on VALFOR® Zeolite 861B.

Said zeolites are preferably impregnated with said specified metals as their salts, particularly their metal nitrate or chloride salts, in an aqueous, alcoholic, or ketonic media over a period of 1-24 hours, then the solids are filtered off, dried at elevated temperature, e.g. 120°C, for a period of time and calcined at 300-800°C for a further period, e.g. 315°C for 2 hours, followed by 540°C for another 2 hours.

Example 1 demonstrates the preparation of the multimetal-modified catalysts. Salts of iron, chromium and manganese, such as their chlorides or nitrates, in anhydrous or hydrated forms were dissolved in water, alcohol, or acetone and the β-zeolites were added, most often, in the form of extrudates. The catalysts were then calcined by heating to 300° to 800°C and optionally reduced in a stream of hydrogen at 200°C.

The amount of the various metals deposited on the zeolite can vary. The amount of each individual metal, iron, chromium, copper, manganese, and nickel, can vary from 0.01 to 5.0%. Where iron, chromium and manganese are deposited on 861β the preferred weight percent is from 0.01% to 5.0%.

Said catalysts may be in the form of powders, pellets, granules, spheres, shapes and extrudates. The examples described herein demonstrate the advantages of using extrudates.

The reaction may be carried out in either a stirred slurry reactor or in a fixed bed continuous flow reactor. The catalyst concentration should be sufficient to provide the desired catalytic effect. Said catalysts may be formed in the presence of a binder, such as Group III or Group IV oxide, including alumina or silica. Said binders may comprise 10% to 90% of the formed catalyst.

Etherification can generally be conducted at temperatures from 20° to 250°C; the preferred range is 80° to 200°C. Good results are observed throughout this temperature range. However, it can be noted that the best conversion figures for DTBP and tert-butanol are observed when the temperature is around 140°C or higher. The total operating pressure may be from 0.1 to 7.0 MPa (0 to 1000 psig) or higher. The preferred pressure range is 0.4 to 3.4 MPa (50 to 500 psig).

Typically, MTBE is generated continuously in up to ca. 50 wt% concentration or greater in the crude liquid product at total liquid hourly space velocities (LHSV) of up to 6 or higher and relatively mild conditions, where:

$$\text{LHSV} = \frac{\text{Volume Of Total Liquid Feed Run Through The Reactor Per Hour}}{\text{Volume of Catalyst In Reactor}}$$

Conversions of t-butanol (tBA, wt%) are estimated in the following examples using the equation:

$$\frac{(\text{Mole\% of tBA in Feed - Mole\% of tBA in Product})}{\text{Mole\% of tBA in Feed}} \times 100$$

The examples which follow illustrate the one-step synthesis of MTBE from tBA and MeOH (Eq. 2) using β-zeolites or multimetal-modified β-zeolites particularly in the form of extrudates. The examples are only intended as a means of illustration and it is understood the invention is not meant to be limited thereby.

The accompanying Examples illustrate:

1) The cosynthesis of MTBE, isobutylene ($C_4H_8$) and diisobutylene ($C_8H_{16}$) in Example 2 from t-butanol (tBA)/methanol (MeOH) via etherification, dehydration and dimerization reactions using an iron, chromium, manganese-modified β-zeolite prepared by the method of Example 1. Here tBA conversion levels are typically 62% at 120°C and product phase separation is realized at temperatures of 140°C or above.

2) In Examples 3-8 the cosynthesis of MTBE, isobutylene and diisobutylene from tBA/MeOH is illustrated using:

a) A series of other Fe, Cr, Mn-modified β-zeolites with different metal loadings and different proportions of β-zeolite to alumina.

b) A series of β-zeolites with different zeolite to alumina weight ratios.

In these examples, tBA conversion levels may reach 74% per pass at 120°C (see Example 3) and again crude product effluent phase separation into an isobutylene/MTBE/diisobutylene rich phase and a heavier aqueous methanol phase may be achieved at operating temperatures of 140°C or greater (e.g. Example 8).

3) Examples 9 and 10 illustrate the cosynthesis of MTBE and isobutylene via methanol/t-butanol/etherification using a crude feedstock also containing sizeable quantities of water, MTBE, isopropanol, peroxide, t-butyl formate and diisobutylene. The solid acid catalysts was either β-zeolite or a multi metal-modified β-zeolite. Extended catalyst life has been confirmed for both classes of zeolite.

**EXAMPLE 1**

This example illustrates the preparation of a multimetal-modified β-zeolite.

To 102g of β-zeolite (Valfor C861β, 80% β-zeolite, 20% alumina) in 1.6mm (1/16") diameter extruded form was added a solution of ferric chloride hydrate (1.04g), chromium(III) nitrate, hydrate (1.64g) and manganese(II) nitrate hydrate (1.10g) in 92cc of distilled water. Impregnation of the β-zeolite was allowed to occur over 1 - 2 hours, then the solids were filtered off, dried at 120°C overnight, and calcined at 315°C for 2 hours, followed by 540°C for another 2 hours.

The recovered green solid extrudates showed the presence of:

%Fe = 0.27
%Cr = 0.19
%Mn = 0.08
Acidity = 0.35 meq/g

**EXAMPLE 2**

This example illustrates the production of methyl t-butyl ether from t-butanol and methanol using the Fe, Cr, Mn - impregnated β-zeolite of Example 1.

Synthesis was conducted in a tubular reactor (12.7mm int. diam., 305mm long) constructed of 316 stainless steel, operated upflow, and mounted in a furnace, controllable to ± 1.0°C, and fitted with pumps allowing flow control to ± 1 cc/hr. The reactor was also fitted with a pressure regulating device and equipment for monitoring temperature, pressure, and flow rate.

The reactor was charged at the beginning of the experiment with 25cc of Fe, Cr, Mn - treated β-zeolite, prepared by the procedure of Example 1, as 1.6mm diameter extrudates. A screen of glass wool was placed at the top and bottom of the reactor to ensure the catalyst would remain in the middle portion.

The catalyst bed was treated with a methanol/t-butanol (1.1:1 molar mix) upflow, at a rate of 50cc/hr, while the reactor was held at 120°C, with a total pressure of 2.1 MPa (300psi). Samples of crude product effluent were collected periodically on stream, in 316ss bombs, and analyzed by glc.

Typical analyses data for samples taken under these conditions are summarized in Table 1. Concentrations of MTBE, isobutylene, methanol and t-butanol in the product effluent were also measured at a series of higher temperatures (140-180°C). Those data are also included in Table 1.

For sample #2, at 120°C:

tBA conversion = 62%
MTBE selectivity = 70%
isobutylene selectivity = 19%

For sample #7, at 180°C:
tBA conversion = 97%

TABLE I

| Ex. | Catalyst | MeOH/tBA Molar Ratio | Feed Rate (cc/hr) | Temp. (°C) | Time On Stream (Days) | Sample | Product Composition (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 2 | Ex. 1 | 1.1:1 | 50 | | | | 0.1 | 31.3 | | 68.4 | | |
| | | | | 120 | 1 | 1<br>→2 | 12.7<br>12.9 | 20.2<br>19.5 | 6.0<br>6.1 | 26.3<br>26.0 | 34.5<br>35.2 | N.D.*<br>N.D. |
| | | | | 140 | 2 | 3 | {4.1<br>25.8} | 15.3<br>37.4 | 15.1<br>2.3 | 15.0<br>16.3 | 50.2<br>17.7 | 19.9<br>3.3 |
| | | | | | | 4 | {5.0<br>27.5} | 16.4<br>39.5 | 15.0<br>2.0 | 13.6<br>15.2 | 49.6<br>15.5 | N.D.<br>N.D. |
| | | | | 160 | 3 | 5 | {1.7<br>32.6} | 12.7<br>51.7 | 27.6<br>0.7 | 4.9<br>7.9 | 52.1<br>6.2 | 20.4<br>0.9 |
| | | | | | | 6 | {4.0<br>32.5} | 16.5<br>52.1 | 25.4<br>0.8 | 5.1<br>7.5 | 48.3<br>6.6 | N.D.<br>N.D. |
| | | | | 180 | 4 | →7 | {3.7<br>34.8} | 11.8<br>57.7 | 65.5<br>1.5 | 1.5<br>3.2 | 16.3<br>2.4 | 29.0<br>0.6 |
| | | | | | | 8 | {4.9<br>35.4} | 13.5<br>56.6 | 62.3<br>1.7 | 3.4<br>3.0 | 15.2<br>2.6 | N.D.<br>N.D. |

*Not Determined

Bracketed compositions are those where a two-phase product was observed.

**EXAMPLES 3 - 8**

Using the equipment and following the procedures of example 2, various multimetal-impregnated β-zeolite catalysts and various β-zeolite samples with different levels of alumina binder were treated with a 1.1:1 molar mix of methanol and t-butanol at a series of operating temperatures from 120° to 180°C. Concentrations of MTBE, isobutylene, methanol and t-butanol in the product effluents, under the specified conditions, as determined by glc for each catalyst, are summarized in the accompanying Tables 2 to 7 of particular note:

a) Examples 3 and 4 illustrate, in Tables 2 and 3, the performance of two other Fe, Cr, Mn- modified β-zeolite catalysts, where, for example, in the case of Example 3, 1%Fe, 1%Cr, 1%Mn were impregnated into a 30% β-zeolite, 70% Alumina Matrix (Table 2):

at 120°C, tBA conversion = 58%
MTBE selectivity = 74%
Isobutylene selectivity = 22%
while at 180°C, tBA conversion = 94%

b) Examples 5 through 8 illustrate, in Tables 4 - 7, the performance of β-zeolite with various levels of alumina binder. In the case of the 30% β-zeolite, 70% alumina matrix of Example 7 (Table 6):

at 120°C, tBA conversion = 74%

MTBE selectivity = 75%

Isobutylene selectivity = 17%

while at 180°C, tBA conversion = 94%

TABLE II

| Ex. | Catalyst | MeOH tBA Molar Ratio | Feed Rate (cc/hr) | Temp. (°C) | Time On Stream (Days) | Sample | PRODUCT COMPOSITIOON (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 3 | 052-92-6887-102[a] | 1.1:1 | 50 | | | FS-1 | | 31.4 | | 68.4 | | |
| | | | | 120 | 1 | →1<br>2 | 10.4<br>10.7 | 19.1<br>19.2 | 6.8<br>6.6 | 28.4<br>29.0 | 35.1<br>34.3 | 0.4<br>0.4 |
| | | | | 140 | 2 | 3<br>4 | 12.6<br>12.4 | 19.6<br>19.4 | 9.6<br>10.5 | 22.0<br>21.5 | 35.5<br>35.8 | 1.6<br>1.5 |
| | | | | 160 | 3 | 5 | {3.3<br>32.9 | 15.4<br>38.9 | 33.9<br>5.0 | 11.9<br>11.4 | 31.7<br>11.2 | 3.6<br>0.4 |
| | | | | | | 6 | {6.6<br>33.2 | 17.7<br>39.3 | 33.3<br>5.1 | 11.3<br>11.0 | 30.5<br>10.9 | N.D.<br>N.D. |
| | | | | 180 | 4 | →7 | {5.7<br>32.5 | 14.1<br>51.1 | 59.4<br>4.4 | 3.5<br>6.1 | 17.0<br>5.4 | 6.4<br>0.2 |
| | | | | | | 8 | {2.5<br>32.6 | 9.6<br>50.6 | 64.9<br>4.6 | 3.7<br>6.5 | 19.1<br>5.2 | N.D.<br>N.D. |

[a]1%Fe. 1%Cr. 1%Mn on 30% β-zeolite, 70%$Al_2O_3$

FS = Feed Stock

TABLE III

| Ex. | Catalyst | MeOH/ tBA Molar Ratio | Feed Rate (cc/hr) | Temp. (°C) | Time On Stream (Days) | Sample | PRODUCT COMPOSITION (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 4 | 052-92-6887-002* | 1.1:1 | 50 | | | | | 31.1 | | 68.7 | | |
| | | | | 120 | 1 | 1<br>2 | 11.9<br>11.7 | 17.7<br>17.8 | 6.7<br>6.7 | 29.7<br>29.0 | 33.8<br>34.4 | 1.5<br>1.5 |
| | | | | 140 | 2 | 3<br>4 | 14.1<br>13.4 | 22.0<br>20.5 | 9.1<br>9.3 | 22.7<br>27.0 | 31.8<br>29.6 | 6.8<br>6.6 |
| | | | | 160 | 3 | 5 | 3.3<br>32.2 | 12.4<br>45.5 | 42.4<br>3.5 | 7.2<br>9.4 | 34.2<br>8.9 | 19.8<br>0.9 |
| | | | | | | 6 | 2.5<br>31.4 | 11.7<br>46.0 | 42.1<br>3.7 | 8.9<br>8.8 | 34.5<br>9.4 | N.D.<br>N.D. |
| | | | | 180 | 4 | 7 | 8.7<br>33.4 | 18.6<br>54.8 | 55.2<br>3.0 | 3.2<br>4.7 | 13.9<br>3.5 | 26.8<br>0.5 |
| | | | | | | 8 | 1.3<br>32.7 | 5.7<br>54.4 | 54.6<br>3.4 | 16.9<br>5.0 | 21.2<br>4.1 | N.D.<br>N.D. |

*1%Fe, 1%Cr, 1%Mn On 60% ß-zeolite, 40%$Al_2O_3$

TABLE IV

| Ex. | Catalyst | MeOH tBA Molar Ratio | Feed Rate (cc/hr) | Temp. (°C) | Time On Stream (Days) | Sample | PRODUCT COMPOSITION (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 5 | C861β* | 1.1:1 | 50 | | | FS-1 | 0.1 | 31.3 | | 68.4 | | |
| | | | | 120 | 1 | 1<br>2 | 13.4<br>13.4 | 20.2<br>20.1 | 5.8<br>6.0 | 26.3<br>25.5 | 34.1<br>34.7 | N.D.<br>N.D. |
| | | | | 140 | 2 | 3 | 4.7<br>26.7 | 15.6<br>39.9 | 15.5<br>1.7 | 12.8<br>14.7 | 51.1<br>16.6 | 22.8<br>2.5 |
| | | | | | | 4 | 19.4<br>27.3 | 30.5<br>40.5 | 4.5<br>1.8 | 18.0<br>14.5 | 26.8<br>15.6 | N.D.<br>N.D. |
| | | | | 160 | 3 | 5 | 5.6<br>28.4 | 18.7<br>46.2 | 24.1<br>20.3 | 4.3<br>4.3 | 42.9<br>4.3 | 19.3<br>0.7 |
| | | | | | | 6 | 2.9<br>33.3 | 15.0<br>54.0 | 27.4<br>0.7 | 4.2<br>5.9 | 49.9<br>5.7 | N.D.<br>N.D. |
| | | | | 180 | 4 | 7 | 7.5<br>29.9 | 17.6<br>48.3 | 56.1<br>1.4 | 2.1<br>7.6 | 15.9<br>2.3 | 23.4<br>0.6 |
| | | | | | | 8 | 3.7<br>29.5 | 12.3<br>47.1 | 62.6<br>1.5 | 1.7<br>19.1 | 17.3<br>2.1 | N.D.<br>N.D. |

*β-zeolite from PQ Corp

TABLE V

| Ex. | Catalyst | MeOH tBA Molar Ratio | Feed Rate (cc/hr) | Temp. (°C) | Time On Stream (Days) | Sample | PRODUCT COMPOSITION (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 6 | 090-92-1027-700[a] | 1.1:1 | 50 | | | FS-1 | | 31.3 | | 68.3 | | |
| | | | | 120 | 1 | 1<br>2 | 12.3<br>12.1 | 19.0<br>18.5 | 6.4<br>6.2 | 22.3<br>22.0 | 39.7<br>41.0 | 4.6<br>4.3 |
| | | | | 140 | 2 | 3 | 7.3<br>24.4 | 19.0<br>33.1 | 13.0<br>3.1 | 16.0<br>17.3 | 44.3<br>21.8 | 17.5<br>4.4 |
| | | | | | | 4 | N.D.<br>24.6 | N.D.<br>32.9 | N.D.<br>3.7 | N.D.<br>16.9 | N.D.<br>21.5 | N.D.<br>4.9 |
| | | | | 160 | 3 | 5 | 2.6<br>28.6 | 12.7<br>51.9 | 28.6<br>1.3 | 5.6<br>8.6 | 49.8<br>9.1 | 25.6<br>1.3 |
| | | | | | | 6 | 2.0<br>27.1 | 11.8<br>52.1 | 29.0<br>1.5 | 5.9<br>9.0 | 50.6<br>9.8 | N.D.<br>1.3 |
| | | | | 180 | 4 | 7 | 11.2<br>29.6 | 22.5<br>56.7 | 47.9<br>3.3 | 3.2<br>5.4 | 14.5<br>4.2 | 26.4<br>0.5 |
| | | | | | | 8 | 1.2<br>27.9 | 7.3<br>57.2 | 69.8<br>3.7 | 2.3<br>5.6 | 19.1<br>5.0 | N.D.<br>0.5 |

[a]50% ß-zeolite, 50% $Al_2O_3$

TABLE VI

| Ex. | Catalyst | MeOH tBA Molar Ratio | Feed Rate (cc/hr) | Temp. (°C) | Time On Stream (Days) | Sample | PRODUCT COMPOSITION (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 7 | 090-92-1027-170[a] | 1.1:1 | 50 | | | FS-1 | | 31.3 | | 68.5 | | |
| | | | | 120 | 1 | 1<br>→2 | 11.9<br>11.7 | 18.2<br>18.0 | 6.6<br>6.8 | 21.3<br>17.7 | 41.6<br>45.4 | 2.9<br>2.9 |
| | | | | 140 | 2 | 3/3 | 11.4<br>[b] | 20.2 | 10.7 | 23.1 | 34.3 | 0.6 |
| | | | | | | 4 | 13.7<br>[b] | 24.2 | 9.3 | 15.8 | 36.5 | 10.8 |
| | | | | 160 | 3 | 5 | 2.4<br>27.4 | 11.9<br>46.3 | 34.6<br>2.3 | 7.1<br>11.9 | 43.5<br>11.6 | 26.3<br>1.7 |
| | | | | | | 6 | 2.1<br>26.2 | 11.6<br>47.0 | 34.4<br>3.2 | 7.0<br>12.6 | 44.6<br>10.6 | N.D.<br>1.8 |
| | | | | 180 | 4 | →7 | 3.1<br>27.4 | 10.3<br>54.1 | 64.8<br>4.5 | 3.4<br>6.9 | 17.8<br>6.0 | 17.9<br>0.7 |
| | | | | | | 8 | 0.8<br>29.3 | 6.4<br>54.7 | 71.1<br>3.9 | 2.5<br>7.1 | 18.8<br>4.4 | N.D.<br>0.4 |

[a] 30% ß-zeolite, 70% $Al_2O_3$
[b] Insufficient sample for analysis.

TABLE VII

| Ex. | Catalyst | MeOH/ tBA Molar Ratio | Feed Rate (cc/hr) | Temp. (°C) | Time On Stream (Days) | Sample | PRODUCT COMPOSITION (wt%) | | | | | |
|---|---|---|---|---|---|---|---|---|---|---|---|---|
| | | | | | | | $H_2O$ | MeOH | $C_4H_8$ | tBA | MTBE | $C_8H_{16}$ |
| 8 | 090-92-1017-300[a] | 1.1:1 | 50 | | | FS-1 | | 31.3 | | 68.5 | | |
| | | | | 120 | 1 | 1 | 10.7 | 18.4 | 7.0 | 23.3 | 40.4 | 0.5 |
| | | | | | | 2 | 10.8 | 18.5 | 6.8 | 24.6 | 39.0 | 0.5 |
| | | | | 140 | 2 | 3 | 10.4 | 19.1 | 11.3 | 17.1 | 41.8 | 2.6 |
| | | | | | | 4 | 10.8 | 18.4 | 11.0 | 19.3 | 39.9 | 2.6 |
| | | | | 160 | 3 | 5 | 9.9 | 22.5 | 19.7 | 18.6 | 28.9 | 0.4 |
| | | | | | | 6 | 10.3 | 22.8 | 18.5 | 19.8 | 28.3 | 0.4 |
| | | | | 180 | 4 | 7 | {8.8<br>30.2 | 19.8<br>35.9 | 29.5<br>6.3 | 13.4<br>15.0 | 27.9<br>12.1 | 0.2<br>- |
| | | | | | | 8 | {9.2<br>N.D. | 21.4<br>N.D. | 27.8<br>N.D. | 13.0<br>N.D. | 28.2<br>N.D. | 0.1 |

[a]10% ß-zeolite, 90% $Al_2O_3$

**EXAMPLE 9**

Using the equipment and following the procedures of Example 2, an Fe, Cr, Mn- modified β-zeolite catalyst was treated with a crude, 2:1 molar, mix of methanol to t-butanol feedstock that also contained significant quantities of MTBE, water, isopropanol (2-PrOH), acetone ($Me_2CO$), diisobutylene, methyl ethyl ketone (MEK) di-t-butyl peroxide (DTBP) and t-butyl formate (TBF). Etherification was conducted at 120°C, 2.1 MPa (300psi) using a LHSV of 2.

Concentrations of each of those components, plus isobutylene, and dimethyl ether (DME) in the product effluent were determined by glc. Typical data are given in the accompanying Table 7. Over the 53 day time period of this experiment, there was only a modest change in catalyst activity - as measured by the level of t-butanol conversion. Typical calculated conversion and selectivity data are as follows:

| | | | |
|---|---|---|---|
| Sample : | 1 | 6 | 9 |
| Time of Stream (Days) : | 1 | 33 | 53 |
| t-Butanol Conv. (%) : | 66 | 62 | 58 |
| MTBE Selectivity (Mole%) : | 77 | 80 | 77 |
| Isobutylene Selectivity (Mole%) : | 15 | 17 | 22 |

Product effluent samples typically showed <1 ppm concentrations of iron, chromium and manganese.

TABLE VIII

| Ex. | Catalyst | MeOH tBA Molar Ratio | Temp. (°C) | Time On Stream (Days) | Sample | PRODUCT COMPOSITION (wt%) | | | | | | | | | | |
|---|---|---|---|---|---|---|---|---|---|---|---|---|---|---|---|---|
| | | | | | | $H_2O$ | MeOH | $C_4H_8$ | $Me_2CO$ | 2-PrOH | tBA | MEK + MTBE | DME | TBF | $C_8H_{16}$ | DTBP |
| 9 | 2165-CT-91[a] | 2:1 | | | FS-1 | 5.8 | 38.1 | | 0.6 | 6.4 | 46.3 | 1.5 | - | 0.17 | 0.1 | - |
| | | | 120 | 1 | →1 | 14.9 | 28.1 | 3.5 | 0.7 | 6.6 | 15.7 | 29.5 | 0.03 | - | 1.3 | 0.01 |
| | | | | 4 | 2 | 14.7 | 29.0 | 3.5 | 0.7 | 6.5 | 15.5 | 30.0 | 0.02 | - | 1.1 | 0.01 |
| | | | | 12 | 3 | 13.9 | 27.5 | 3.5 | 0.6 | 6.1 | 20.1 | 28.0 | 0.01 | - | 0.6 | 0.01 |
| | | | | | FS-2 | 5.8 | 38.8 | | 0.6 | 6.3 | 46.3 | 1.7 | - | 0.18 | 0.1 | - |
| | | | | 18 | 4 | 14.2 | 28.8 | 3.6 | 0.6 | 6.4 | 17.3 | 28.9 | 0.01 | - | 0.5 | 0.01 |
| | | | | 25 | 5 | 13.5 | 29.0 | 3.6 | 0.8 | 6.3 | 19.0 | 27.8 | 0.01 | - | - | 0.03 |
| | | | | | FS-3 | 5.8 | 39.0 | | 0.6 | 6.3 | 46.4 | 1.8 | - | 0.20 | 0.1 | - |
| | | | | 33 | →6 | 13.3 | 29.1 | 3.7 | 0.7 | 6.4 | 17.8 | 28.9 | - | - | 0.3 | - |
| | | | | | FS-4 | 5.8 | 39.0 | | 0.6 | 6.3 | 46.0 | 2.1 | - | 0.17 | 0.1 | - |
| | | | | 39 | 7 | 13.2 | 30.2 | 3.7 | 0.6 | 6.4 | 19.1 | 26.7 | - | 0.01 | 0.2 | 0.02 |
| | | | | 47 | 8 | 11.8 | 29.4 | 4.0 | 0.6 | 5.9 | 18.7 | 26.3 | - | 0.01 | 0.2 | 0.02 |
| | | | | | FS-5 | 5.7 | 39.0 | | 0.6 | 6.4 | 46.5 | 1.8 | - | 0.17 | 0.1 | 0.02 |
| | | | | 53 | →9 | 11.7 | 30.3 | 4.4 | 0.6 | 6.1 | 19.5 | 26.6 | - | - | 0.2 | 0.01 |

[a] 1% Cr, 1%Mn, 1% Fe ON VALFOR C8612β

**EXAMPLE 10**

Following the procedures of Example 2, the β-zeolite catalyst of Example 5 was treated with the same crude 2:1 molar mix of methanol/t-butanol of Example 9. Etherification was conducted at 120°C, 2.1 MPa (300 psi), using a LHSV of 2

Concentrations of each component was followed by glc analyses of the product effluents. Typical data are given in the accompanying Table 9. Over the 21 day time period of this experiment, there was no loss in catalyst activity - as measured by the level of t-Butanol conversion. Typical calculated conversion and selectivity data are as follows:

| | | |
|---|---|---|
| Sample : | 1 | 4 |
| Time of Stream (Days) : | 1 | 21 |
| t-Butanol Conv. (%) : | 71 | 73 |
| MTBE Selectivity (Mole%) : | 67 | 80 |
| Isobutylene Selectivity (Mole%) : | 14 | 19 |

TABLE IX

| Run | Catalyst | MeOH/ tBA Molar Ratio | Temp. (°C) | Time On Stream (Days) | Sample | PRODUCT COMPOSITION (wt%) | | | | | | | | | | |
|---|---|---|---|---|---|---|---|---|---|---|---|---|---|---|---|---|
| | | | | | | $H_2O$ | MeOH | $C_4H_8$ | $Me_2CO$ | 2-PrOH | tBA | MEK + MTBE | DME | TBF | $C_8H_{16}$ | DTBP |
| 6945-46 | C861β* | 2:1 | | | FS-1 | 5.9 | 38.8 | | 0.6 | 6.3 | 46.2 | 2.1 | - | 0.19 | - | - |
| | | | 120 | 1 | →1 | 15.9 | 31.6 | 3.5 | 0.7 | 6.8 | 13.2 | 28.3 | 0.11 | - | 4.5 | - |
| | | | | 7 | 2 | 15.0 | 30.5 | 3.6 | 0.7 | 6.6 | 13.8 | 29.7 | 0.07 | 0.01 | 3.2 | - |
| | | | | | FS-2 | 5.6 | 39.2 | | | 6.3 | 46.2 | 1.9 | - | 0.19 | - | - |
| | | | | 15 | 3 | 13.5 | 28.9 | 4.3 | 0.7 | 6.2 | 13.6 | 32.0 | 0.04 | 0.01 | 1.6 | - |
| | | | | | FS-3 | 5.8 | 39.0 | | 0.6 | 6.3 | 46.4 | 1.7 | - | 0.17 | 0.1 | 0.02 |
| | | | | 21 | →4 | 12.9 | 27.7 | 4.9 | 0.6 | 5.9 | 12.3 | 34.3 | 0.02 | - | 1.0 | - |

* β-zeolite from PQ Corp

**Claims**

1. A process for the preparation of alkyl tertiary alkyl ether from tertiary alkyl alcohol and primary alcohol in the presence of a catalyst, characterised in that the catalyst is a β-zeolite or a β-zeolite modified with one or more metals selected from Groups IB, VB, VIB, VIIB, VIII of the Periodic Table.

2. A process for the preparation of methyl tert-butyl ether from t-butanol and methanol in the presence of a catalyst, characterised in that the catalyst is a β-zeolite or a β-zeolite modified with one or more metals selected from Groups IB, VB, VIB, VIIB, VIII of the Periodic Table.

3. A process as claimed in Claim 2, wherein said t-butanol and methanol are present in a molar amount of 0.1 to 10 moles of methanol per mole of t-butanol.

4. A process as claimed in Claim 2 or 3 wherein said t-butanol and methanol are continuously contacted with said catalyst at a temperature of 20°C to 250°C and a pressure of 0.1 to 7.0 MPa to obtain methyl tert-butyl ether.

5. A process as claimed in any one of Claims 1 to 4 wherein the β-zeolite has a silica:alumina ratio of 10:1 to 100:1.

6. A process as claimed in any one of Claims 1 to 5 wherein the β-zeolite contains $Na_2O$ 0.5%-1.0% by weight anhydrous.

7. A process as claimed in any one of Claims 1 to 6 wherein the β-zeolite is modified with one or more metals selected from the group consisting of iron, chromium, manganese, copper and nickel.

8. A process as claimed in any one of Claims 1 to 7 wherein the concentrations of metals deposited on said zeolite may vary from 0.01% to 5.0% for each metal.

9. A process as claimed in any one of Claims 1 to 8 wherein the β-zeolite catalyst is formed in the presence of a Group III or Group IV oxide.

10. A process as claimed in any one of Claims 2 to 9 wherein the operating temperature is in the range 140°C to 200°C and the product comprises a two-phase mix of an isobutylene-MTBE product-rich phase and a heavier aqueous methanol-rich phase.

**Patentansprüche**

1. Ein Verfahren zur Herstellung von Alkyl-tert.-alkylether aus tertiärem Alkylalkohol und primärem Alkohol in der Gegenwart eines Katalysators, dadurch gekennzeichnet, daß der Katalysator ein β-Zeolith oder ein β-Zeolith, der mit einem oder mehreren Metallen modifiziert ist, die ausgewählt sind aus den Gruppen IB, VB, VIB, VIIB, VIII des Periodensystems, ist.

2. Ein Verfahren zur Herstellung von Methyl-tert.-butylether aus tert.-Butanol und Methanol in der Gegenwart eines Katalysators, dadurch gekennzeichnet, daß der Katalysator ein 9-Zeolith oder ein β-Zeolith, der mit einem oder mehreren Metallen der modifiziert ist, die ausgewählt sind aus den Gruppen IB, VB, VIB, VIIB, VIII des Periodensystems, ist.

3. Ein Verfahren nach Anspruch 2, wobei besagtes tert.-Butanol und Methanol in einer molaren Menge von 0,1 bis 10 Mol Methanol pro Mol tert.-Butanol vorhanden sind.

4. Ein Verfahren nach Anspruch 2 oder 3, wobei besagtes tert.-Butanol und Methanol kontinuierlich mit besagtem Katalysator bei einer Temperatur von 20°C bis 250°C und einem Druck von 0,1 bis 7,0 MPa in Kontakt gebracht werden, um Methyl-tert.-butylether zu erhalten.

5. Ein Verfahren nach einem der Ansprüche 1 bis 4, wobei der β-Zeolith ein Silciumdioxid:Aluminiumoxid-Verhältnis von 10:1 bis 100:1 hat.

6. Ein Verfahren nach einem der Ansprüche 1 bis 5, wobei der β-Zeolith $Na_2O$ zu 0,5-1,0 Gew.-% wasserfrei enthält.

7. Ein Verfahren nach einem der Ansprüche 1 bis 6, wobei der β-Zeolith mit einem oder mehreren Metallen modifiziert ist, die ausgewählt sind aus der Gruppe, die aus Eisen, Chrom, Mangan, Kupfer und Nickel besteht.

8. Ein Verfahren nach einem der Ansprüche 1 bis 7, wobei die Konzentrationen der Metalle, die auf besagtem Zeolith abgeschieden sind, von 0,01% bis 5,0% für jedes Metall variieren können.

9. Ein Verfahren nach einem der Ansprüche 1 bis 8, wobei der β-Zeolithkatalysator in der Gegenwart eines Oxids der Gruppe III oder Gruppe IV gebildet ist.

10. Ein Verfahren nach einem der Ansprüche 2 bis 9, wobei die Betriebstemperatur im Bereich von 140°C bis 200°C liegt und das Produkt ein Zwei-Phasen-Gemisch aus einer an Isobutylen-MTBE-Produkt reichen Phase und einer schwereren, wäßrigen, an Methanol reichen Phase umfaßt.

**Revendications**

1. Procédé pour la préparation d'éther d'alkyle et de tertioalkyle à partir d'alcool tertioalkylique et d'alcool primaire en présence d'un catalyseur, caractérisé en ce que le catalyseur est une β-zéolite ou une β-zéolite modifiée avec un ou plusieurs métaux choisis parmi les groupes IB, VB, VIB, VIIB, VIII du tableau périodique.

2. Procédé pour la préparation d'éther de méthyle et de tert-butyle à partir de t-butanol et de méthanol en présence d'un catalyseur, caractérisé en ce que le catalyseur est une β-zéolite ou une β-zéolite modifiée avec un ou plusieurs métaux choisis parmi les groupes IB, VB, VIB, VIIB, VIII du tableau périodique.

3. Procédé selon la revendication 2, dans lequel lesdits t-butanol et méthanol sont présents en une quantité molaire de 0,1 à 10 moles de méthanol par mole de t-butanol.

4. Procédé selon la revendication 2 ou 3, dans lequel lesdits t-butanol et méthanol sont mis en contact en continu avec ledit catalyseur à une température de 20 °C à 250 °C et une pression de 0,1 à 7,0 MPa pour obtenir de l'éther de méthyle et de tert-butyle.

5. Procédé selon l'une quelconque des revendications 1 à 4, dans lequel la β-zéolite a un rapport silice:alumine de 10:1 à 100:1.

6. Procédé selon l'une quelconque des revendications 1 à 5, dans lequel la β-zéolite contient $Na_2O$ 0,5 % à 1,0 % en poids anhydre.

7. Procédé selon l'une quelconque des revendications 1 à 6, dans lequel la β-zéolite est modifiée avec un ou plusieurs métaux choisis dans le groupe constitué du fer, du chrome, du manganèse, du cuivre et du nickel.

8. Procédé selon l'une quelconque des revendications 1 à 7, dans lequel les concentrations des métaux déposés sur ladite zéolite peuvent varier de 0,01 % à 5,0 % pour chaque métal.

9. Procédé selon l'une quelconque des revendications 1 à 8, dans lequel le catalyseur β-zéolite est formé en présence d'un oxyde du groupe III ou du groupe IV.

10. Procédé selon l'une quelconque des revendications 2 à 9, dans lequel la température opératoire est dans la gamme de 140 °C à 200 °C et le produit comprend un mélange biphasique, d'une phase riche en produit isobutylène-MTBE et d'une phase aqueuse plus lourde riche en méthanol.